# FASCICULE DE BREVET EUROPEEN

(11) **EP 0 542 941 B1**
(45) Date de publication et mention de la délivrance du brevet: **22.01.1997**
(21) Numéro de dépôt: 92908293.1
(22) Date de dépôt: 27.04.1992
(51) Int. Cl.: A61K 7/44

(54) **LES N-PHENYL-BENZAMIDES PROTECTEURS CONTRE LES EFFETS NOCIFS DE LA LUMIERE ULTRA-VIOLETTE**
N-PHENYL-BENZAMIDE ZUM SCHUTZ VOR DEN SCHÄDLICHEN WIRKUNGEN VON ULTRAVIOLETTLICHT
N-PHENYL-BENZAMIDES PROVIDING PROTECTION FROM THE HARMFUL EFFECTS OF ULTRAVIOLET LIGHT

(30) Priorité: 26.04.1991 CH 1249/91
(43) Date de publication de la demande: 26.05.1993
(73) Titulaire: BAUDET, Pierre, CH-1234 Vessy (CH)
(72) Inventeur: BAUDET, Pierre, CH-1234 Vessy (CH)
(86) Numéro de dépôt international: CH9200082
(87) Numéro de publication internationale: WO9219208

(56) Documents cités:
- EP-A- 0 232 199
- CH-A- 342 700
- US-A- 2 874 090
- US-A- 3 175 950
- US-A- 3 863 007
- PATENT ABSTRACTS OF JAPAN vol. 12, no. 391 (C-537)(3238) 18 Octobre 1988

## Description

La lumière solaire, au niveau de l'écorce terrestre, comprend une partie de lumière ultra-violette dont le spectre s'étend de 280 à 400 nm. Les radiations de longueur d'onde plus courte sont retenues par la couche d'ozone circumterreste, dans la haute atmosphère. La lumière ultra-violette des courtes longueurs d'ondes à 280 nm est appelée UV-C, de 280 à 320 nm UV-B et de 320 à 400 nm UV-A.

L'irradiation d'un organisme vivant par les UV-C est léthale; l'irradiation par les UV-B est toxique, provoquant de l'érythème et des lésions du stratum corneum: l'irradiation par les UV-A provoque le brunissement de la peau en induisant la mélanogenèse dans les mélanocytes. La mélanine formée à partir de la dopamine provenant de la tyrosine est transférée dans le kératocytes. Le brunissement rend l'épiderme moins sensible à l'effet toxique des UV-A qui pénétrent dans le derme. Il n'empêche cependant pas les UV-A de provoquer le vieillissement de la peau et l'induction de carcinomes cutanés.

La quantité de UV-A parvenant sur la surface terrestre est env.1000 fois plus importante que la quantité de UV-B.

L'organisme humain, en particulier les téguments (peau et cheveux) doit être protégé contre les effets néfastes des radiations UV-A et UV-B de la lumière solaire et celà d'autant plus que la population est de race blanche et qu'elle vit sur des continents à forte exposition solaire ou qu'elle s'expose momentanément à de fortes irradiations solaires.

Pour réaliser cette protection on a créé des produits cosmétiques, appelés filtres ou écrans solaires, contenant des substances inorganiques ou organiques qui réfléchissent ou absorbent respectivement la lumière UV-A et UV-B. Les substances minérales sont le talc, l'oxyde de zinc et l'oxyde de titane, on les appelle des écrans solaires.

On leurs préfére pour l'esthétique des solutions translucides ou laiteuses de molécules organiques, les filtres solaires, en solution dans des crèmes, des laits, des lotions, des huiles, des gels, à raison d'une concentration de 0,5 à 10 %. Les molécules organiques sont choisies en fonction de leur pouvoir d'absorption de la lumière ultra-violette UV-A et UV-B et de leur inocuité.

Dans le tableau I nous présentons les principaux filtres solaires commercialisés, en indiquant leur longueur d'onde maximum de leur spectre d'absorption UV A et UV-B (λ max.) et l'intensité de leur pouvoir d'absorption des UV-A et UV-B, exprimé en coefficient d'extinction moléculaire (ε max.), leur appartenance à la classe des UV.

**Tableau I**

| filtres | λ max. (nm) | ε max | UV |
|---|---|---|---|
| 1) acide para-amino-benzoique | 283 | 15300 | B |
| 2) para-diméthylamino-benzoate d'octyle | 311 | 27300 | B |
| 3) 4-méthoxy-2-hydroxy-benzophénone | 288 | 14000 | B |
| | 325 | 9400 | A |
| 4)4-méthoxy-2-hydroxy-3-sulfo-benzophénone | 286 | 13400 | B |
| | 325 | 8400 | A |
| 5) 2,2'-dihydroxy-4-méthoxy-benzophénone | 284 | 13270 | B |
| | 327 | 10440 | A |
| 6) 4-méthoxy-cinnamate de 2-éthyl-héxyle | 311 | 23300 | B |
| 7) 4-méthoxy-4'-n-butyl-dibenzoyl-méthane | 358 | 34720 | A |
| solvant: éthanol | | | |

Les préparations cosmétiques contiennent presque toujours un mélange de filtres UV-A et UV-B, afin de réaliser la plus large protection possible.

Les qualités idéales des filtres solaires UV-A et UV-B sont les suivantes:
1) une absorbabilité de la lumière UV-A et UV-B supérieure à ε max.20000,
2) une bonne photostabilité,
3) une bonne stabilité chimique,
4) une bonne fixation au stratum corneum,
5) une bonne incorporation à la composition cosmétique,
6) une bonne inocuité,
7) ne pas tacher.

La présente invention concerne des substances organiques utilisables en qualité de filtre solaires, d'écrans solaires, de protecteurs des denrées alimentaires et des matériaux contre les altérations provoquées par la lumière ultra-violette.

La structure de ces substances organiques a été choisie en fonction
1) de leur grand pouvoir absorbant la lumière UV-A et UV-B
   (ε max suprérieur à 20000),
2) de leur photostabilité,
3) de leur parentée à des substances déjà utilisées comme filtres solaires, dont on a reconnu à l'échelle mondiale, leur inocuité sanitaire.
4) leur résidtivité en milieu aqueux,
5) leur appartenance à ume même famille de composés dont de faible variation permet de moduler leurs propriétés UV-A et UV-B.

Une bonne absorbabilité des lumières UV-A et UV-B requiert de la part de la substance organique un degré d'insaturation qui augmente en passant des structures UV-B aux structures UV-A.

La modulation des propriétés absorbantes, exprimées en ε max., requiert aussi la présence de fonctions organiques à effet inductif (donneur d'électrons) sur le système insaturé, par ex. les fonctions alkoxy, hydroxy, amino, dialkylamino, et en position favorable par rapport à ces fonctions des groupements d'atomes attracteurs d'électrons, par ex. les fonction-carboxy, carbo-alkoxy, carboxamide.nitrile, cétonique, sulfonique.

Les structures insaturées au carbone, non cyclique, par ex. les dérivés de l'éthylène, subissent l'isomérie cis-trans dont souven l'équilibre est déplacé par la lumière ultra-violette vers l'isomère géomètrique au pouvoir absorbant le plus bas. Par contre, une fonction comprenant deux centre polarisables entre deux restes aromatiques est susceptible de créer une conjugaison dont l'état excité par les photons ultra-violets pourrait être d'énergie relativement peu élevée par rapport à l'état fondamental. Cet effet provoquera selon les fonctions présentes un déplacemements hypsochromique ou bathochromique, voire auxochromique des valeurs spéctrales c-à-d permettra le choix d'une structure appropriée pour la préparation d'un filtre solaire souhaité.

Nous avons choisi pour la fonction intermédiaire, polarisable, entre deux restes aromatiques, la fonction -CONH- réalisant des N-phényl-benzamides. La classe des N-phénylbenzamides est bien connue; elles peuvent s'appeler également N-benzoyl-anilides. Les N-phénylbenzamides qui peuvent être citées en conjonction avec celles que nous décrivons dans cette demandes sont des N-( 4-(alkyl)ou(alkoxy)ou(chloro) ou (fluoro) ou (trifluorométhyl)-4-dialkylamini-benzamides qui sur le reste phényle ne portent pas de fonction accepteur d'électrons. Ces benzamides absorbent les UV-B (US Patent 3,863,007 (28.01.1975) Warner, Jr.) La N-(carboxy-phényl)-4-amino-benzamide (comme sel de bases organiques), la N-(4-carboxy-phényl)-4-acétylamino -beuzamide (comme sels de bases organiques) sont décrites dans CH-A-342 700 et leurs utilisations comme filtres solaires UV-B revendiquée. De nombreuses N-phényl-benzamides sont revendiquées comme filtres solaires UV-B dans US Patent 3,175,950 . La N-(4-hydroxy-phényl)-2-hydroxy-benzamide est décrite dans US Patent 2,874,090. Cette benzamide absorbe dans les UV-B.

Des N-phényl-benzamides, précurseurs de benzophénone sont citées dans Patent Abstracts of Japan, vol 12, no 391(C-537)(3238); elles absorbent les UV-B. Des N-(4-carbo-alkoxy-phényl)-4-alkoxy-benzamides sont revendiqués pour le traitement de désordres de la kératogenèse (Ep. 0 232 199 A2.)

Les pouvoirs d'absorption UV des N-phényl-benzamides de la présente invention se situent entre 280 et 350 nm et leurs intensités d'absorption sont trés élevées. La position de leur λ max peut être déterminée en fonction du choix des groupements donneurs d'électrons sur un cycle aromatique et accepteurs d'électrons sur l'autre cycle aromatique. POur la raison de la meilleure transmission des effets électroniques à travers toute la molécule. nous préférons les positiops para et ortho définies par rapport à la place de la fonction -CONH- intermédiaire entre les deux cycles aromatiques.

Les quatre structures générales des N-phényl-benzamides de l'invention sont représentées dans le tableau II,

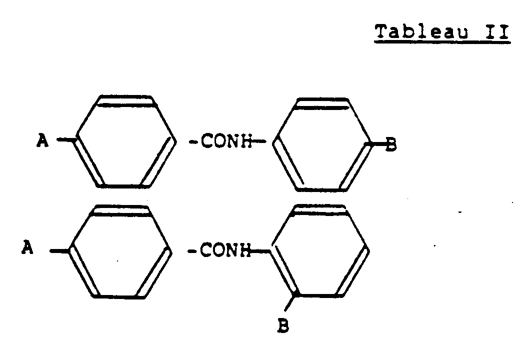

Lorsque à partir de la fonction donneur d'électron A, la fonction B accepteur d'électron permet d'étendre la polarisation à l'autre reste aromatique, l'effet bathochromique s'accroit (déplacement dans les UV-A); lorsque la fonction A est moins donneur d'électrons l'effet hypsochromique se manifeste ( déplacement dans les UV-B).

Ainsi, dans la même famille de substances, les N-phényl-benzamides, on peut confectionner un filtre solaire à trés fort pouvoir absorbant de la lumière ultra violette choisie. Ces propriétés conférent à ces N-phényl-benzamides, comme filtres solaires, une qualité de protection que l'on ne connaît pas chez les filtres solaires commercialisés (voir tableau I).

Dans le tableau III nous présentons les valeurs de λ max. et de ε max. pour une série de N-phényl-benzamides de l'invention, en fonction de la nature des substituants A et B,

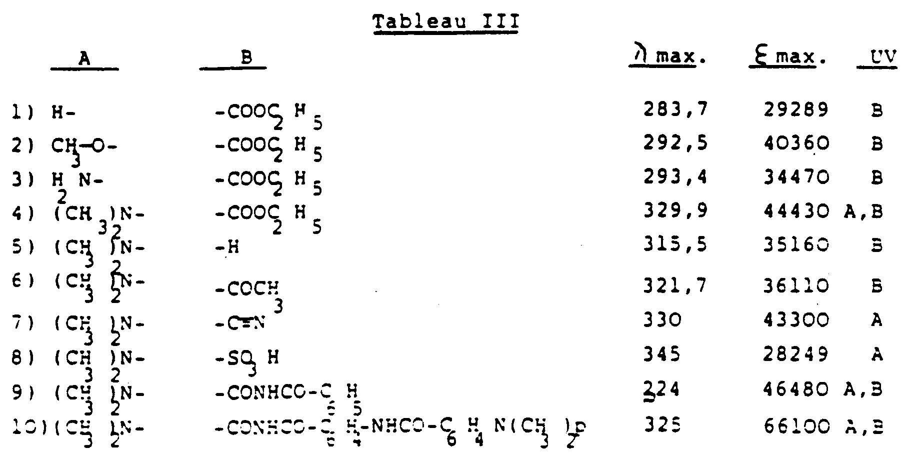

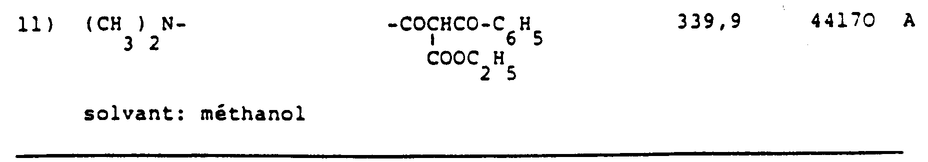

La protection excercée par un filtre solaire s'èvaluant d'abord par la valeur de son coefficient d'extinction moléculaire (ε) dans UV-A et UV-B, nous donnons dans le tableau IV les ε des N-phénylbenzamides, N-(4-(N-benzoyl-carboxamido-phényl)-4-diméthylamino-benzamide, N-(4-benzoyl-malonyl éthyl ester-phényl)-4-diméthylamino-benzamide en solution, ensemble dans le méthanol (10⁻⁵ M).

**Tableau IV**

| λ max (nm) | ε max | λ max.(nm) | ε max. |
|---|---|---|---|
| 280 | 41000 | 340 | 74500 |
| 290 | 46000 | 350 | 52000 |
| 300 | 55000 | 360 | 32000 |
| 310 | 66000 | 370 | 14920 |
| 320 | 78000 | | |
| 330 | 73000 | | |

Dans le tableau V nous présentons les valeurs de ε max pour une solution dans le méthanol (10⁻⁵ M) de N-(4-carboéthoxy-phényl)-4-diméthylamino-benzamide et de N-(4-sulfo-phényl)-4-diméthylamino-benzamide.

**Tableau V**

| λ max. (nm) | ε max. | λ max. (nm) | ε max. |
|---|---|---|---|
| 280 | 30970 | 330 | 68000 |
| 290 | 30500 | 340 | 60000 |
| 300 | 36000 | 350 | 42500 |
| 310 | 48000 | 360 | 29900 |
| 320 | 60250 | 370 | 18300 |

La photostabilité des N-phényl-benzamides à la lumière ultra-violette a été déterminée par irradiation des solutions dans le méthanol des produits , à une concentration de 10⁻⁵ M, dans des cuvettes en quartz de 10mm. Ces solutions sont exposées sur un plateau tournant à la lumière d'une lampe Theimer Metallhalgenidstrhahler Violight 510 820197. Les durées d'exposition ont été de 15, 30, 45, 60 et 90 min., aprés quoi les spectres ultra-violets sont réexaminés.

Les N-phényl-benzamides se sont révélées photostables.

### Les exemples:

La synthèse des N-phényl-benzamides a été effectuée à partir des dérivés appropriés de l'acide benzoique dont la fonction carboxy a été transformée en chlorure d'acide ou anhydride d'acide ou en estersdit activé, ou en carbazide ou en anhydride mixte ou en dérivé de carbodiimide réagissant avec la fonction amino-phényl substitué ou non, dans un solvant anhydre. Les produits sont cristallisé dans de l'eau puis recristallisé comme indiqué pour chaque N-phényl-benzamide décrite.

Les analyses ont portés sur la composition C, H, N, S; les point de fusion déterminé par l'appareil de Kofler (Reichert), les spectres ultra-violets ont été déterminé avec le spectrophotomètre Cary 2300 dans du méthanol, avec des solutions 10⁻⁵ M de 500 à 200 nm; les spectres IR ont été obtenus avec un spectrophotomètre 735 B de Perkin-Elmer

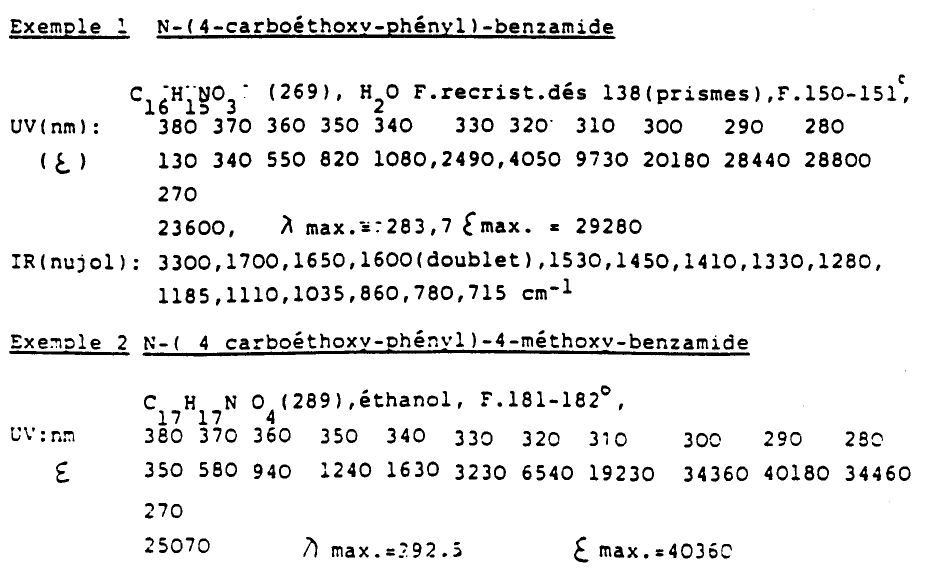

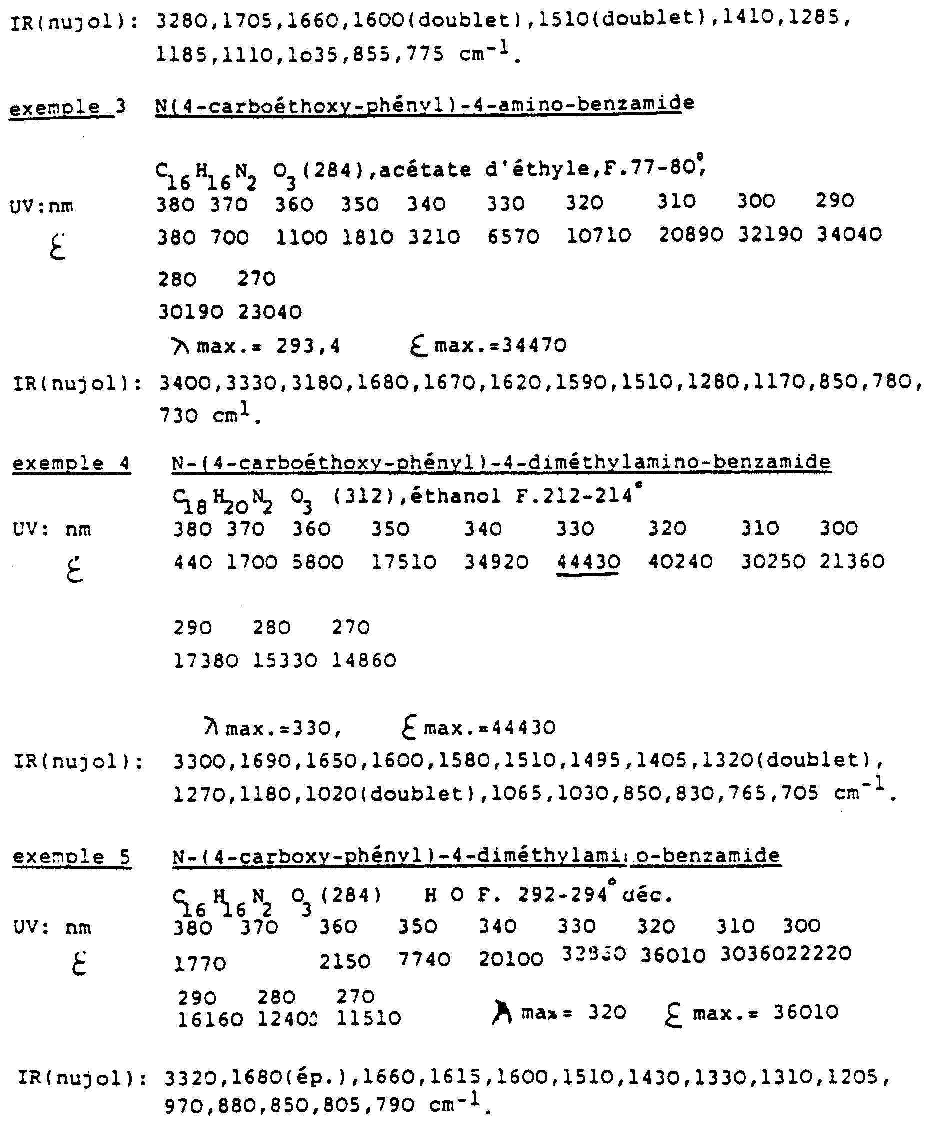

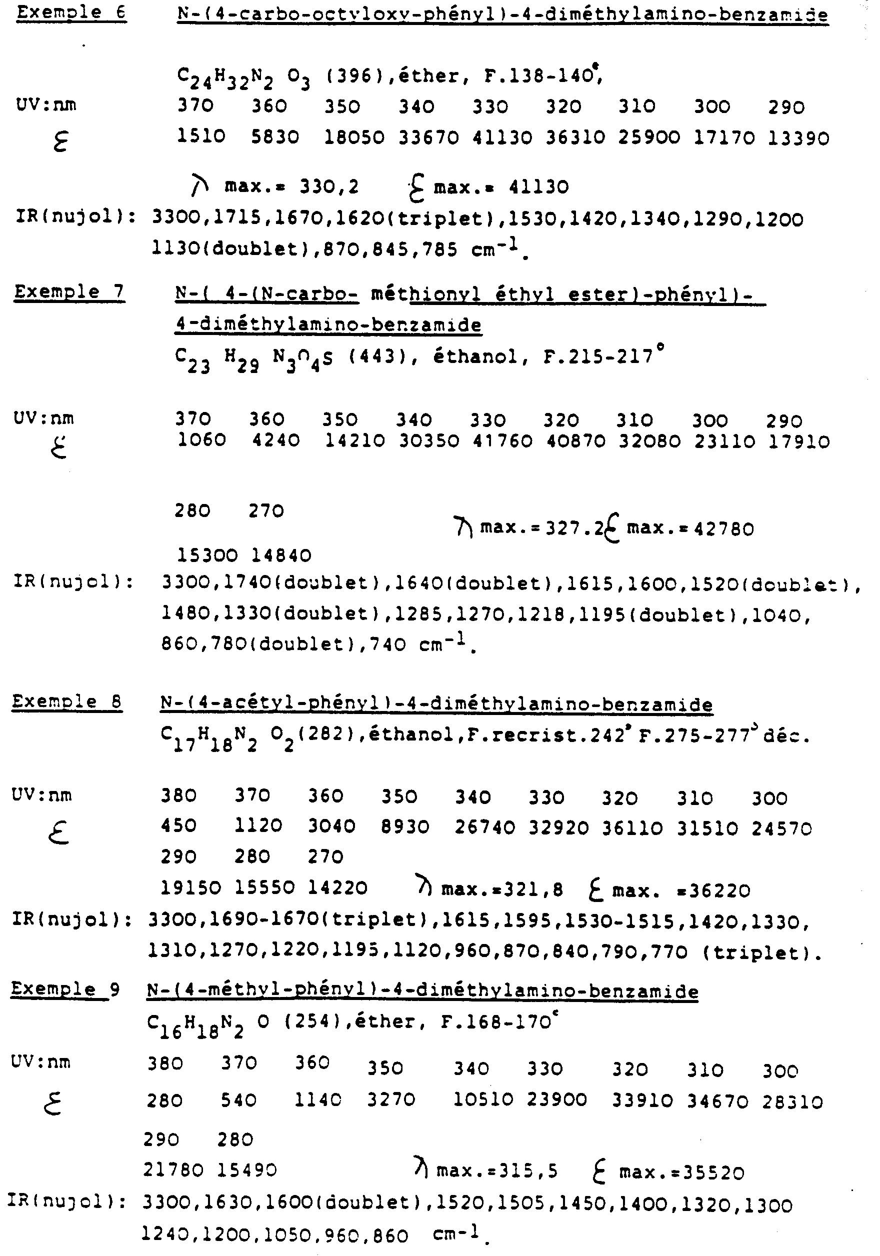

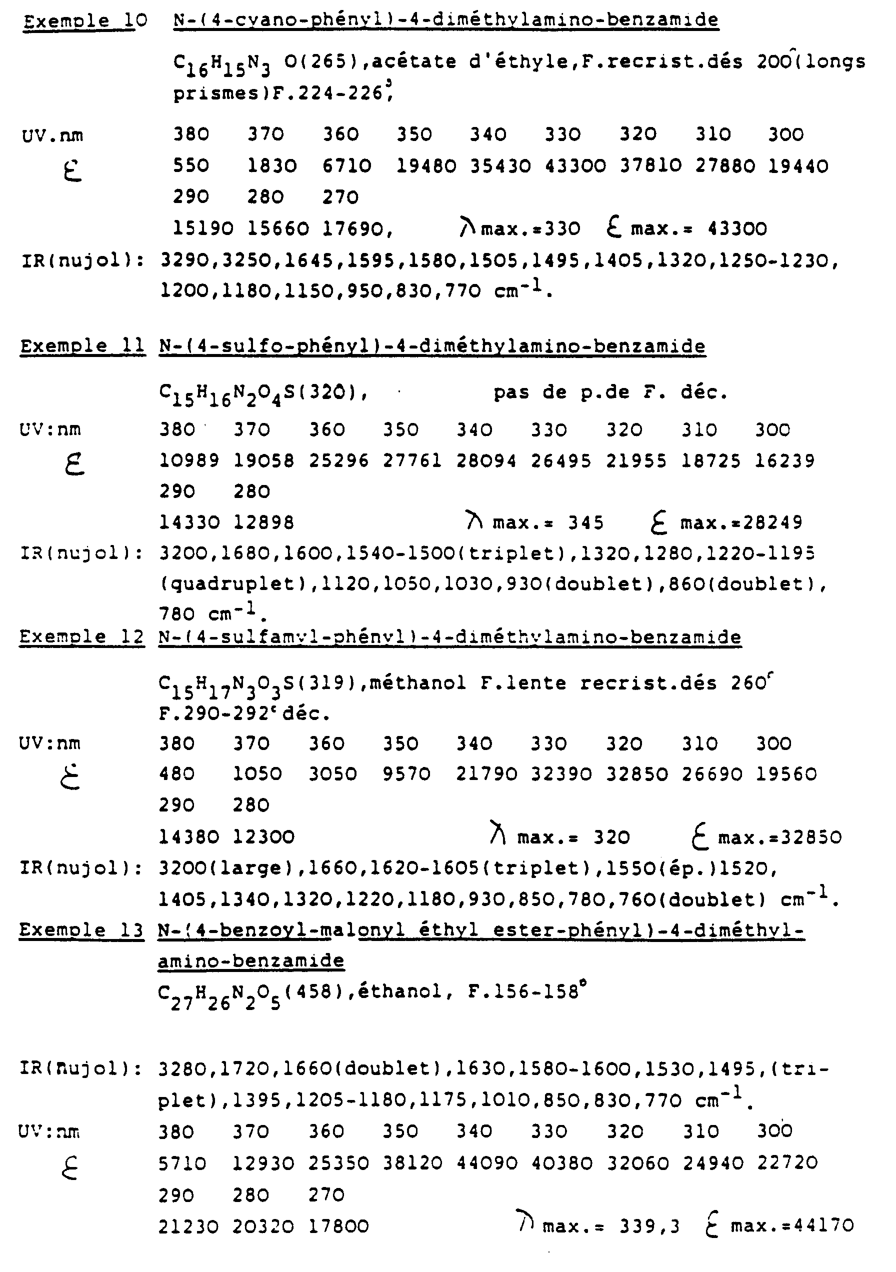

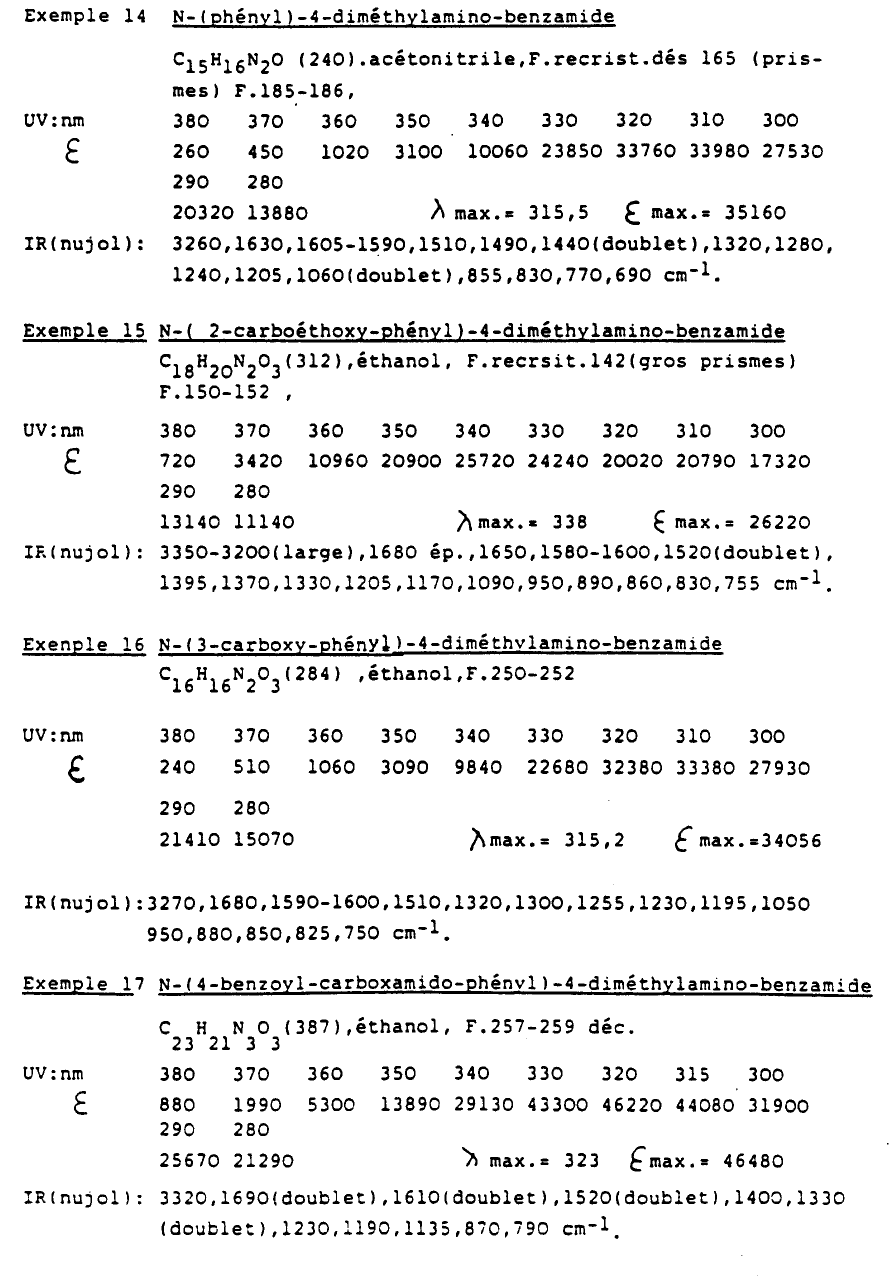

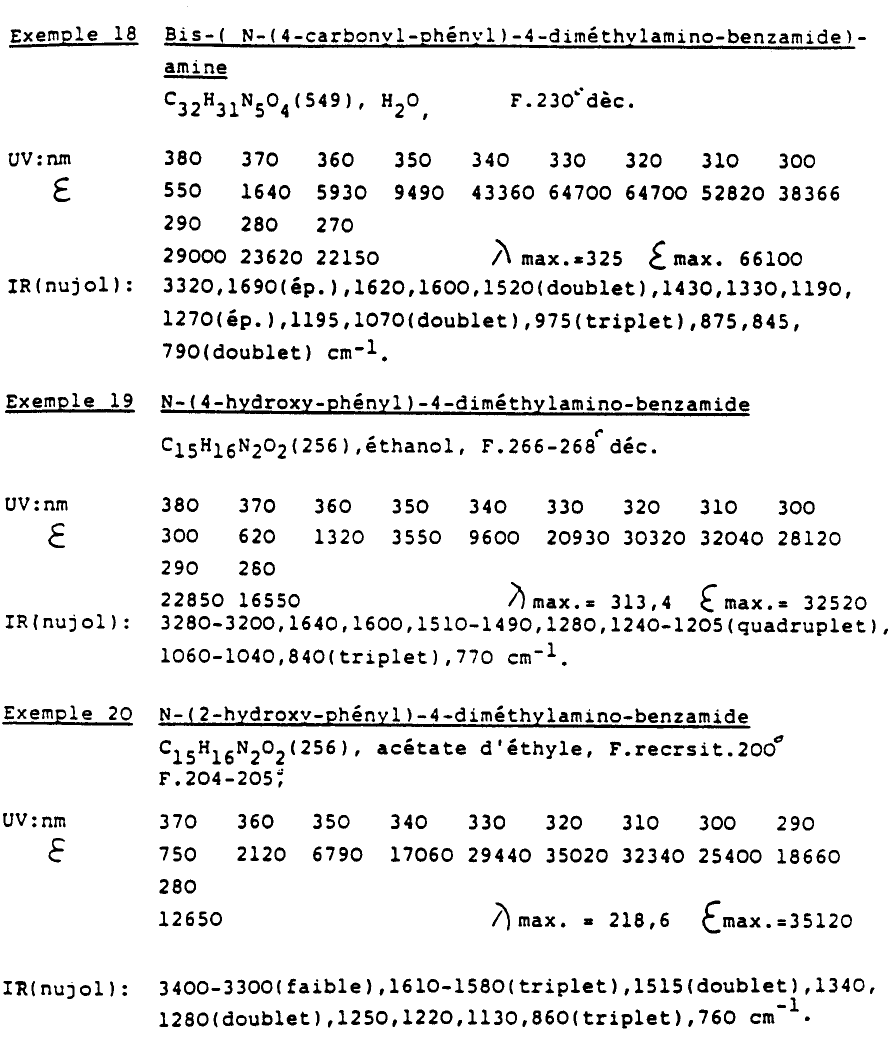

## Revendications

1. N-phényl-benzamides de la formule I, utilisés en qualité de filtres solaires et d'écrans solaires en cosmétiques, utilisés en qualité d'agents de protection des denrées alimentaires contre les dommages provoqués par la lumière ultra-violette, utilisés en qualité d'agents de protection des matériaux contre les dégradations dues à la lumière ultra-violette. 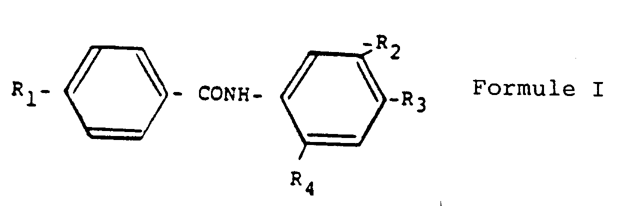 dans laquelle R₁ est une fonction mono-alkyl-amino, une fonction dialkylamino, une fonction alkoxy, une fonction phénoxy.
dans laquelle R₂ est un hydrogène, une fonction hydroxy, une fonction acide carboxylique, une fonction ester carboxylique, une fonction alkoxy dont le reste alkyle a 1-6 atomes de carbone, une fonction sulfonique,
dans laquelle R₃ est une fonction acide carboxylique, une fonction ester carboxylique, une fonction carboxamide, une fonction amide avec un acide aminé, une fonction amide peptidique, une fonction amide avec une amine aromatique, une fonction amide acylée, une fonction nitrile, une fonction cétonique aliphatique, une fonction cétonique aromatique, une fonction di-cétonique,
une fonction sulfo, une fonction sulfamyle, une fonction sulfoxide, une fonction sulfone, un reste 2-benzimidazole, un reste 2-benzothiazole,
dans la quelle R₄ est un hydrogène, une fonction hydroxy, une fonction alkoxy dont le reste alkyle a 1-6 atomes de carbone. Les autres positions aromatiques du reste phényle sur lequel est placé R₁ peuvent être substituées par des fonctions ou des restes utiles à l'usage proposé.

2. N-phényl-benzamide selon la revendication 1 de la formule II, caractérisée par ses propriétés de filtre solaire A et B, de protecteur des denrées alimentaires et des matériaux contre les dommages provoqués par la lumière ultra-violette,

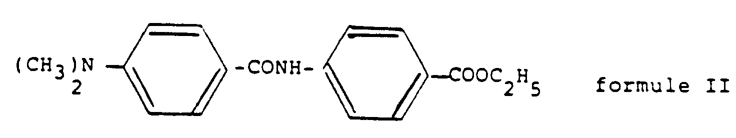

3. N-phényl-benzamide selon la revendication 1 de la formule IV, caractérisée par ses propriétés de filtre solaire A et B, de protecteur des denrées alimentaires et des matériaux contre les dommages provoqués par la lumière ultra-violette,

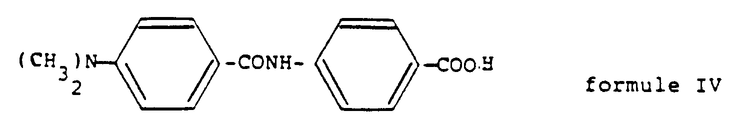

4. N-phényl-benzamide selon la revendication 1 de la formule V, caractérisée par ses propriétés de filtre solaire UV-A et UV-B, de protecteur des denrées alimentaires et des matériaux contre les dommages provoqués par la lumière ultra-violette,

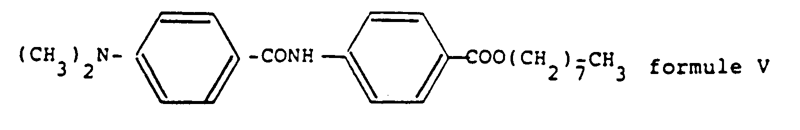

5. N-phényl-benzamide selon la revendication 1 de la formule VI, caractérisée par ses propriétés de filtre solaire UV-A et UV-B, de protection des denrées alimentaires et des matériaux contre les dommages provoqués par la lumière ultra-violette,

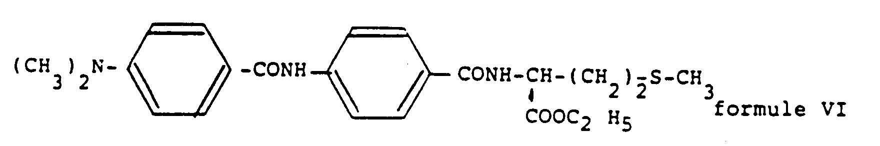

6. N-phényl-benzamide selon la revendication 1 de la formule VII, caractérisée par ses propriétés de filtre solaire UV-A, de protection des denrées alimentaires et des matériaux contre les dommages provoqués par la lumière ultra-violette,

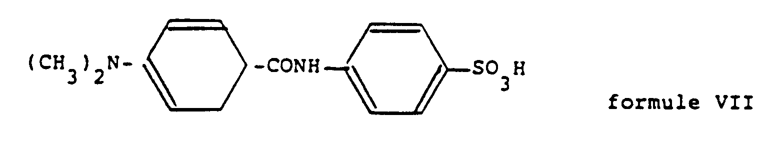

7. N-phényl-benzamide selon la revendication 1 de la formule VIII, caractérisée par ses propriétés de filtre solaire UV-A et UV-B, de protection des denrées alimentaires et des matériaux contre les dommages provoqués par la lumière ultra-violette,

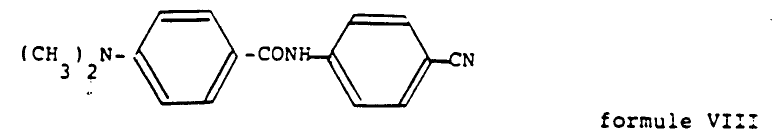

8. N-phényl-benzamide de la formule IX, selon la revendication 1, caractérisée par ses propriétés de filtre solaire UV-A, de protection des denrées alimentaires et des matériaux contre les dommages provoqués par la lumière ultra-violette,

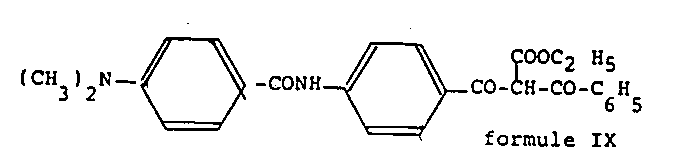

9. N-phényl-benzamide de la formule X, selon la revendication 1, caractérisée par ses propriétés de filtre solaire UV-A et UV-B, de protection des denrées alimentaires et des matériaux contre les dommages provoqués par la lumière ultra-violette,

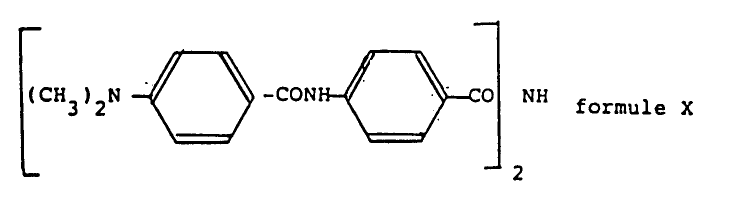

10. N-phényl-benzamide de la formule XI, selon la revendication 1, caractérisée par ses propriétés de filtre solaire UV-A et UV-B, de protection des denrées alimentaires et des matériaux contre les dommages provoqués par la lumière ultra-violette,

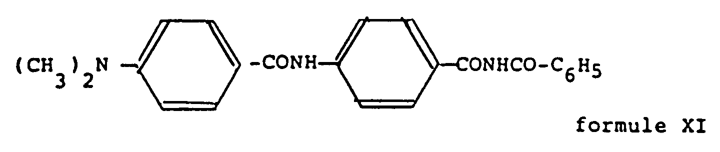

11. N-phényl-benzamide de la formule XII, selon la revendication 1, caractérisée par ses propriétés de filtre solaire UV-A, de protection des denrées alimentaires et des matériaux contre les dommages provoqués par la lumière ultra-violette,

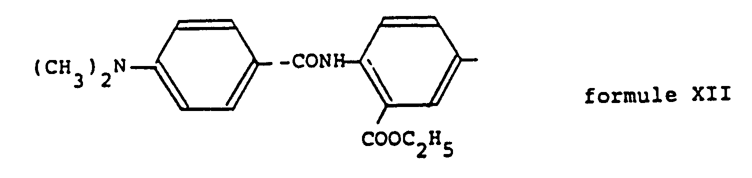

12. N-phényl-benzamide de la formule XIII, selon la revendication 1, caractérisée par ses propriétés de filtre solaire UV-A, de protection des denrées alimentaires et des matériaux contre les dommages provoqués par la lumière ultra-violette,

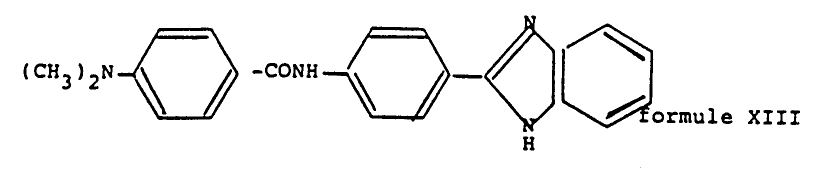

13. Usage de N-phénylbenzamides selon les revendications 1-12 en qualité de filtres solaires UV-AB pour des applications dans des formulations cosmétiques.

14. Usage de N-phénylbenzamides selon les revendications 1-12 en qualité d'agents de protection des denrées alimentaires contre les dommages provoqués par la lunière ultra-violette.

15. Usage de N-phénylbenzamides selon les revendications 1-12 en qualité d'agents de protection des matériaux contre les dommages provoqués par la lumière ultra-violette.

## Claims

1. N-phenyl-benzamides having the formula I, used as a cosmetic solar filters and sunscreens, and as agents protecting food products and material from damage caused by ultra-violet light 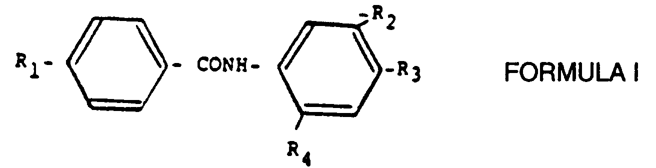 Wherein R1 is selected from mono-alky-amino function, dialkyamino function, alkoxy function, phenoxy function
Wherein R2 is selected from a hydrogen, a hydroxy function, a carboxylic acid function, a carboxylic ester function, an alkoxy function of up to 1-6 carbons, a sulfonic acid function
Wherein R3 is selected from a carboxylic acid function, a carboxylic ester function, a carboxamide function, a carboxamide function with amino-acid, a peptidic carboxamide function, a carboxamide with an aromatic amine, a N-acylated amide function, a nitrite funtion, an aliphatic ketone function, an aromatic ketone function, a di-ketone function, a sulfonic acid function, a sulfamyl function, a sulfoxide function, a sulfo function, 2-benzimidazole residue, a 2-benzothrazole residue
in wherein R4 is selected from an hydrogen, an hydroxy function, an alkoxy function of up to 6 carbons
the other aromatic positions of phenyl residue having R1 can be substituted by other functions or other residues usefull for proposed use

2. N-phenyl-benzamide, follower claim 1, having formula II, characterised by his solar filter A and B properties, as agent protecting food products and materials from damage caused by ultra-violet light

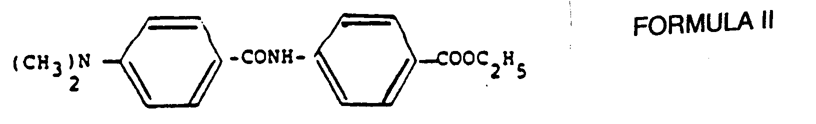

3. N-phenyl-benzamide, follower claim 1, having formula IV, characterised by his solar filter A and B properties, as agent protecting food products and materials from damage caused by ultra-violet light

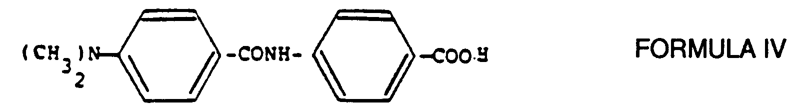

4. N-phenyl-benzamide, follower claim 1, having formula V, characterised by his solar filter A and B properties, as agent protecting food products and materials from damage caused by ultra-violet light

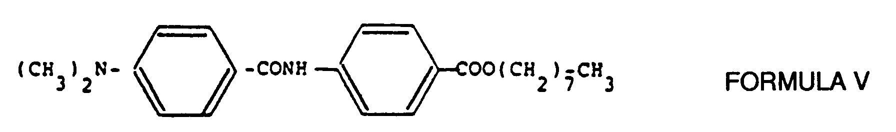

5. N-phenyl-benzamide, follower claim 1, having formula VI, characterised by his solar filter A and B properties, as agent protecting food products and materials from damage caused by ultra-violet light

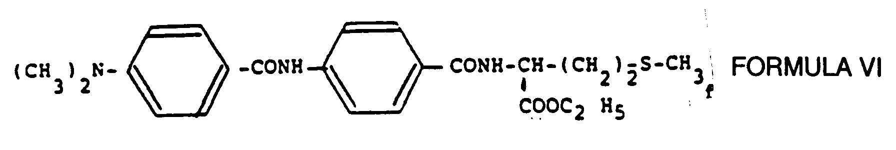

6. N-phenyl-benzamide, follower claim 1, having formula VII, characterised by his solar filter A properties, as agent protecting food products and materials from damage caused by ultra-violet light

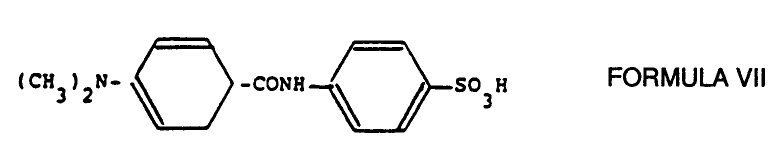

7. N-phenyl-benzamide, follower claim 1, having formula VIII, characterised by his solar filter A and B properties, as agent protecting food products and materials from damage caused by ultra-violet light

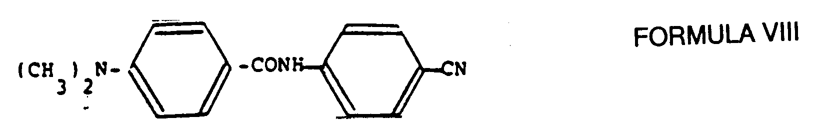

8. N-phenyl-benzamide, follower claim 1, having formula IX, characterised by his solar filter A properties, as agent protecting food products and materials from damage caused by ultra-violet light

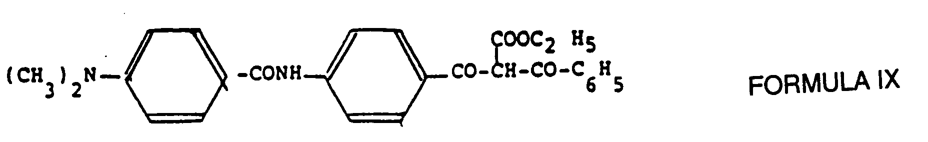

9. N-phenyl-benzamide, follower claim 1, having formula X, characterised by his solar filter A and B properties, as agent protecting food products and materials from damage caused by ultra-violet light

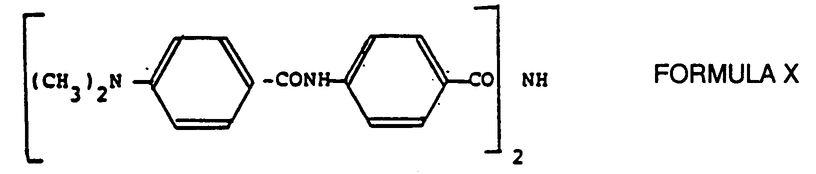

10. N-phenyl-benzamide, follower claim 1, having formula XI, characterised by his solar filter A and B properties, as agent protecting food products and materials from damage caused by ultra-violet light

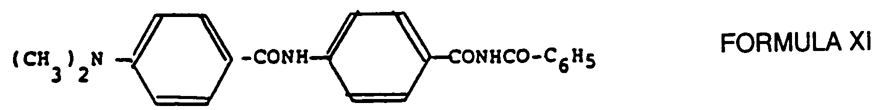

11. N-phenyl-benzamide, follower claim 1, having formula XII, characterised by his solar filter A properties, as agent protecting food products and materials from damage caused by ultra-violet light

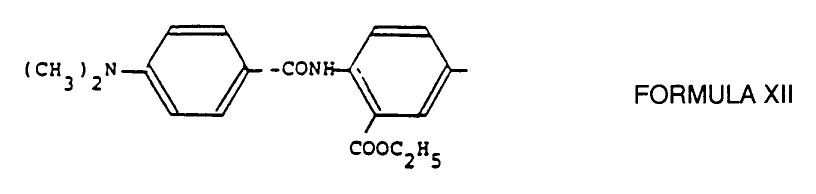

12. N-phenyl-benzamide, follower claim 1, having formula XIII, characterised by his solar filter A properties, as agent protecting food products and materials from damage caused by ultra-violet light

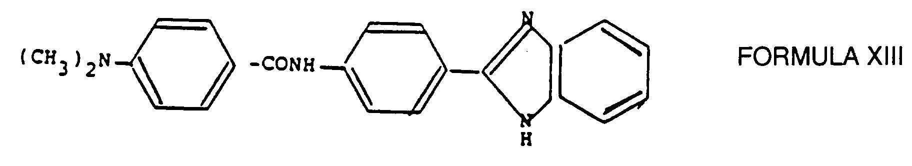

13. Use, following claims 1 to 12 of the N-phenyl-benzamides, as sunscreens UV AB, for use in cosmetic formulations

14. Use, following claims 1 to 12 of the N-phenyl-benzamides, as agent protecting food products from damage caused by ultra-violet light

15. Use, following claims 1 to 12 of the N-phenyl-benzamides, as agent protecting materials from damage caused by ultra-violet light

## Patentansprüche

1. N-phenyl Benzamide der Formel I, als Sonnenfilter und als Sonnenschutz in der Kosmetik gebraucht, als Schutzmittel für Lebensmittel gegen durch ultraviolettes Licht hevorgerufene Schäden gebraucht, als Schutzmittel für Materialien gegen durch ultraviolettes Licht hervorgerufene Degradierungen. 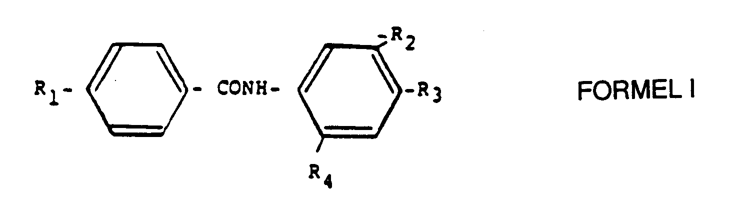 wo R1 eine *Monoalkylaminfunktion, eine Dialkylaminfunktion, eine Alkoxyfunktion, eine Phenoxyfunktion* ist.
wo R2 ein *Wasserstoff, eine Hydroxyfunktion, eine Carbonsäurefunktion, eine Carbonsäureesterfunktion, eine Alkoxyfunktion dessen Alkylrest aus 1 bis 6 Kohlenstoffatomen besteht, eine Sulfonsäurefunktion* ist.
wo R3 eine *eine Carbonsäurefunktion, eine Carbonsäureesterfunktion, eine Carboxamidfunktion, eine Amidfunktion mit einer Aminosäure, eine peptidische Amidfunktion, eine Amidfunktion mit einem aromatischen Amin, eine acylierte Amidfunktion, eine Nitrilfunktion, eine aliphatische Ketonfunktion, eine aromatische Ketonfunktion, eine Sulfofunktion, eine Sulfamylfunktion, eine Sulfoxydfunktion, eine Sulfonfunktion, ein 2-Benzimidazolrest, ein 2-Benzothiazolrest* ist.
wo R4 ein *Wasserstoff, eine Hydroxyfunktion, eine Alkoxyfunktion dessen Alkylrest aus 1 bis 6 Kohlenstoffatomen besteht, eine Sulfonsäurefunktion* ist.
Die anderen aromatischen Positionen des Phenylrestes, auf dem sich R1 befindet, können durch Funktionen und Reste für die vorgeschlagene Anwendung substituiert werden.

2. N-phenyl-benzamide der Formel II gemäss dem Patentanspruch 1, durch ihre Eigenschaften als A und B Sonnenfilter gekennzeichnet, als Schutzmittel von Lebensmitteln und Materialien gegen durch ultraviolettes Licht hervorgerufene Schäden

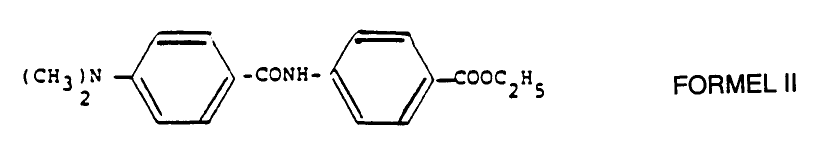

3. N-phenyl-benzamide der Formel IV gemäss dem Patentanspruch 1, durch ihre Eigenschaften als A und B Sonnenfilter gekennzeichnet, als Schutzmittel von Lebensmitteln und Materialien gegen durch ultraviolettes Licht hervorgerufene Schäden,

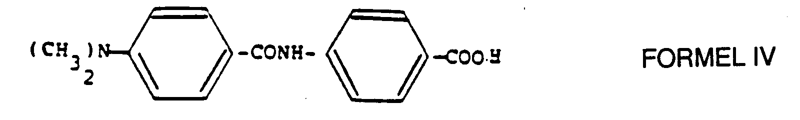

4. N-phenyl-benzamide der Formel V gemäss dem Patentanspruch 1, durch ihre Eigenschaften als UV-A und UV-B Sonnenfilter gekennzeichnet, als Schutzmittel von Lebensmitteln und Materialien gegen durch ultraviolettes Licht hervorgerufene Schäden,

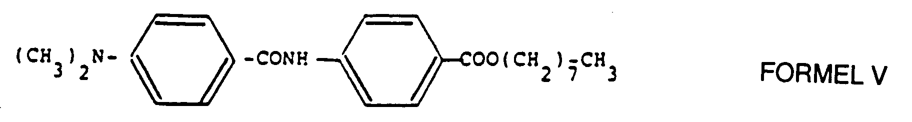

5. N-phenyl-benzamide der Formel VI gemäss dem Patentanspruch 1, durch ihre Eigenschaften als UV-A und UV-B Sonnenfilter gekennzeichnet, als Schutzmittel von Lebensmitteln und Materialien gegen durch ultraviolettes Licht hervorgerufene Schäden,

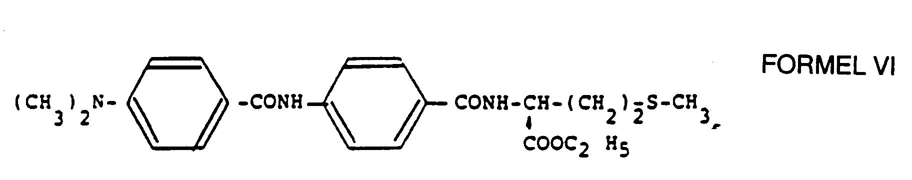

6. N-phenyl-benzamide der Formel VII gemäss dem Patentanspruch 1, durch ihre Eigenschaften als UV-A Sonnenfilter gekennzeichnet, als Schutzmittel von Lebensmitteln und Materialien gegen durch ultraviolettes Licht hervorgerufene Schäden,

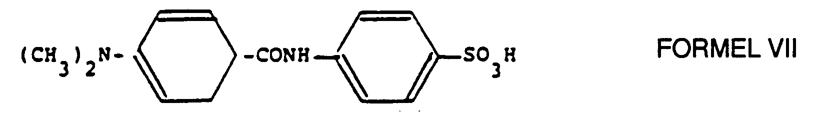

7. N-phenyl-benzamide der Formel VIII gemäss dem Patentanspruch 1, durch ihre Eigenschaften als UV-A und UV-B Sonnenfilter gekennzeichnet, als Schutzmittel von Lebensmitteln und Materialien gegen durch ultraviolettes Licht hervorgerufene Schäden,

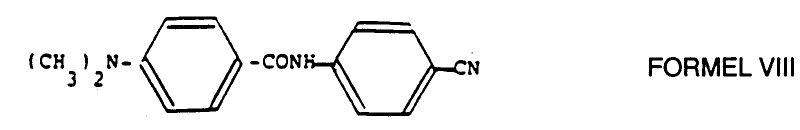

8. N-phenyl-benzamide der Formel IX gemäss dem Patentanspruch 1, durch ihre Eigenschaften als UV-A Sonnenfilter gekennzeichnet, als Schutzmittel von Lebensmitteln und Materialien gegen durch ultraviolettes Licht hervorgerufene Schäden,

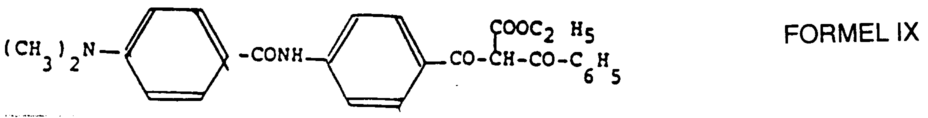

9. N-phenyl-benzamide der Formel X gemäss dem Patentanspruch 1, durch ihre Eigenschaften als UV-A und UV-B Sonnenfilter gekennzeichnet, als Schutzmittel von Lebensmitteln und Materialien gegen durch ultraviolettes Licht hervorgerufene Schäden,

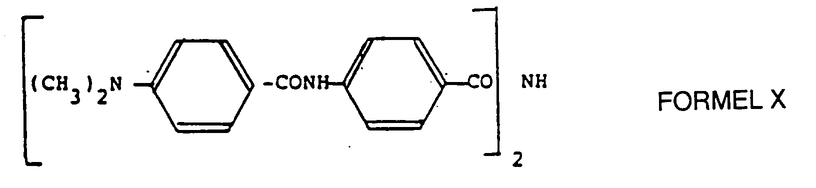

10. N-phenyl-benzamide der Formel XI gemäss dem Patentanspruch 1, durch ihre Eigenschaften als UV-A und UV-B Sonnenfilter gekennzeichnet, als Schutzmittel von Lebensmitteln und Materialien gegen durch ultraviolettes Licht hervorgerufene Schäden,

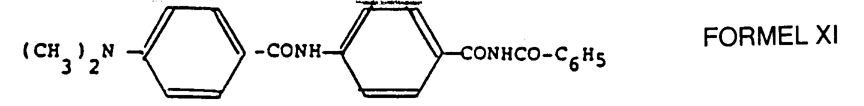

11. N-phenyl-benzamide der Formel XII gemäss dem Patentanspruch 1, durch ihre Eigenschaften als UV-A Sonnenfilter gekennzeichnet, als Schutzmittel von Lebensmitteln und Materialien gegen durch ultraviolettes Licht hervorgerufene Schäden,

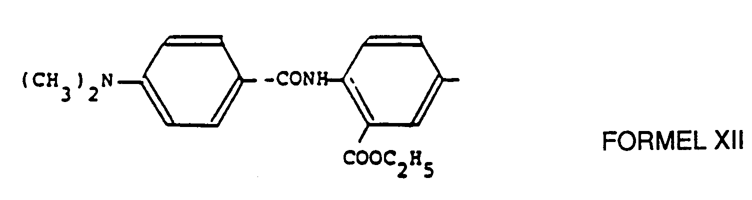

12. N-phenyl-benzamide der Formel XIII gemäss dem Patentanspruch 1, durch ihre Eigenschaften als UV-A Sonnenfilter gekennzeichnet, als Schutzmittel von Lebensmitteln und Materialien gegen durch ultraviolettes Licht hervorgerufene Schäden,

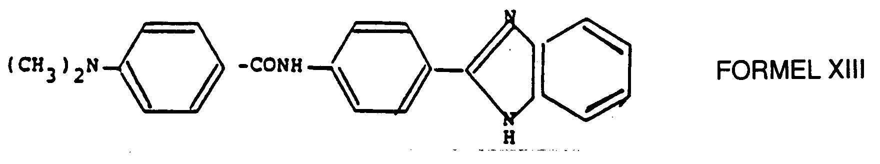

13. Verwendung gemäss den Patentansprüchen 1 bis 12, als sonnenfilter, die für Kosmetische Zwecke nützlich sind,

14. Verwendung gemäss den Patentansprüchen 1 bis 12, als schutzmittel von Lebensmitteln gegen durch ultraviolett Licht hervorgerufene Schäden,

15. Verwendung gemäss den Patentansprüchen 1 bis 12, als Schutzmitteln Materialen hervorgerufene Schäden.
